# EUROPEAN PATENT SPECIFICATION

(11) **EP 1 899 014 B1**
(45) Date of publication and mention of the grant of the patent: **28.09.2011**
(21) Application number: 06765954.0
(22) Date of filing: 29.06.2006
(51) Int. Cl.: A61Q 15/00, A61K 8/02, A61K 8/04, A61K 8/26, A61K 8/28, A61K 8/44, A61K 8/36, A61K 8/362, A61K 8/365, C01G 25/00

(54) **PARTICULATE ENHANCED EFFICACY ANTIPERSPIRANT SALT WITH RAISED PH**
TEILCHENFÖRMIGES ANTIPERSPIRANS-SALZ MIT VERBESSERTER WIRKSAMKEIT UND ERHÖHTEM PH
SEL ANTITRANSPIRANT PARTICULAIRE A EFFICACITE RENFORCEE A PH ACCRU

(30) Priority: 30.06.2005 US 171690
(43) Date of publication of application: 19.03.2008
(73) Proprietor: The Gillette Company, Boston, MA 02199 (US)
(72) Inventor: SHEN, Yanfei, Canton, MA 02021 (US); PROVANCAL, Stephen, J., Elmhurst, IL 60126 (US); ORYSZCZAK, Richard, Palatine, IL 60067 (US)
(74) Representative: Wilding, Richard Alan
(86) International application number: PCT/IB2006/052188
(87) International publication number: WO 2007/004163

(56) References cited:
- EP-A- 0 599 775
- WO-A-96/14052
- WO-A-03/094880
- WO-A2-00/10512
- US-A- 3 991 176
- US-A- 3 998 788
- US-A- 4 774 079
- US-A1- 2004 265 255
- US-B2- 6 749 841

## Description

### Background of the Invention

The present invention relates to a solid particulate enhanced efficacy aluminum-zirconium antiperspirant salt with a pH greater than 4.5.

Enhanced efficacy aluminum-zirconium antiperspirant salts are well known. See, for example, US 4,775,528 (Callaghan) and US 4,871,525 (Giovanniello). The enhanced efficacy salts provide greater sweat reduction than conventional antiperspirant salts and are typically differentiated from conventional antiperspirant salts by reference to the various aluminum peaks that can be identified when the salt is analyzed by size exclusion chromatography, typically HPLC (high pressure liquid chromatography). A suitable chromatographic technique must be capable of resolving the Al into at least four distinct peaks (labeled peaks 2 (or 1+2), 3, 4 and 5), such as is shown in US 5,330,751. The enhanced efficacy salts have been described as having an increased peak 4 content or an increased peak 4 to peak 3 ratio compared to conventional salts. (In some cases, enhanced salts have been described as having increased "band III" content by some authors, depending on the chromatographic technique and nomenclature employed. Generally, bands I, II, III and IV of one system correspond to peaks 1+2 (band I), 3, 4 and 5 of the other system.) Typically, the enhanced efficacy salts (measured as 10% solutions) have an HPLC peak 4 to peak 3 area ratio of 0.5 or higher, preferably at least 0.7, with at least 70%, preferably at least 80%, of the aluminum contained in peaks 3 and 4. Thus, the enhanced salts will typically have a peak 4 content of at least 30% of the total aluminum contained in all the peaks (measured by peak area). In contrast, conventional non-enhanced antiperspirant salts have a negligible peak 4 content or a peak 4 to 3 area ratio less than 0.2, typically about 0.1.

Commercially available solid particulate enhanced efficacy aluminum-zirconium antiperspirant salts are fairly acidic, typically having a pH between about 3 and 4, more typically between about 3.5 and 3.8. Such acidic salts can be somewhat irritating to the skin and, in some cases, can discolor fabric. Such acidic salts also typically have some undesirable odor and can be yellowish in color. In addition, when such salts are formulated into topical compositions, their acidity can cause degradation of other pH-sensitive cosmetic ingredients, such as fragrances, botanicals, etc., that a formulator might desire to include in a topical composition. Some attempts, particularly with respect to non-enhanced salts, have been made to buffer or increase the pH moderately, for example, up to about 4.5, but any further pH increases were believed to reduce efficacy or to possibly cause salt precipitation during manufacture.

In US 4,369,173 (Causland), a non-enhanced aluminum-zirconium chlorohydrex-glycine salt is buffered by encapsulating it in a hydrolyzed carbohydrate such as dextrin or maltrin. However, because the encapsulating material has a pH of about 5.2 or less (see col. 7, lines 1-2), none of the examples provide a salt with a pH above 4.0. (see Tables VII and VIII at col. 17). In US 6,749,841 (Joshi), a gel degradation inhibitor, such as zinc glycinate, is added to an aqueous solution of a non-enhanced aluminum-zirconium chlorohydrex-glycine to increase the pH to 4.2-4.5. This aqueous solution is then mixed with an oil phase to form an emulsion gelled with a polysaccharide. In US 5,997,850 (Tang), a stabilized aluminum-zirconium chlorohydrex-glycine aqueous solution is provided with an increased glycine (or amino acid) content to prevent polymerization of the Zr species. The glycine is added in an amount to provide a Zr:Gly weight ratio of 1:1.2-1:5. This antiperspirant solution is then mixed with an oil phase to form a gelled emulsion (see example 9).

In US 4,774,079 (Shin), non-enhanced antiperspirant compositions are disclosed that comprise a mixture of aluminum chloride, aluminum chlorohydrate and aluminum-zirconium polychlorohydrate complex. The antiperspirant composition is buffered to a pH in the range of 2.5-4.5, preferably 2.8-3.8. All of the examples indicate a pH below 3.8. In US 4,017,599 (Rubino), non-enhanced aluminum-zirconium antiperspirant salts of various types are disclosed. Such salts include an alkali metal or alkaline earth metal salt of an amino acid, such as for example sodium glycinate or magnesium glycinate, to provide a pH of at least about 3. Although this reference suggests a possible pH range of 3 to 5 (see col. 5, lines 9-13), none of the examples that provide pH data indicate a pH above 3.9, which is consistent with the general state of the antiperspirant art.

In US 5,643,558 (Provancal), there is disclosed a polyhydric alcohol solution (for example, a propylene glycol solution) of an enhanced efficacy aluminum-zirconium chlorohydrex antiperspirant salt. This reference suggests that the pH of the polyhydric alcohol solution may be raised to about 4.1-5.0 by addition of an alkaline glycinate, such as sodium, potassium or zinc glycinate. In US 5,463,098 (Giovanniello) discloses a diol soluble aluminum-zirconium chlorohydrex-glycine antiperspirant salt. This salt is prepared by forming an aqueous solution of the salt, to which has been added some propylene glycol and zinc glycinate, the latter to increase the pH to about 4.1-5.0, then spray drying. The dried salt is essentially a salt-diol adduct that may then be redissolved in propylene glycol (or other diols) for use in diol-based gel sticks.

It would be highly desirable to provide a solid particulate antiperspirant salt with a pH greater than 4.5. Such a salt would provide less irritation than current commercially available salts. Such a salt would also permit topical compositions to be formulated with pH-sensitive ingredients, particularly fragrance components that degrade in the highly acidic environment of current commercially available salts. Thus, salts with a pH greater than 4.5 will allow the formulator to choose from a much wider variety of fragrance components and other pH sensitive components.

### Summary of the Invention

The present invention embraces a solid particulate antiperspirant salt comprising a mixture (or complex) of an enhanced efficacy aluminum-zirconium chlorohydrex-amino acid and a neutralizing salt. The particulate anti-perspirant salt, when measured as an aqueous solution at a concentration of 15% by weight at 25ºC, has a pH greater than 4.5, preferably about 4.6 to about 5.3. The present invention also embraces a topical antiperspirant composition comprising the aforementioned particulate antiperspirant salt and a method of reducing perspiration from human skin by applying the afore-mentioned antiperspirant composition. In addition, the present invention includes a method of preparing the aforementioned particulate antiperspirant salt.

### Detailed Description of the Invention

The aluminum-zirconium salts of the present invention are of the enhanced efficacy type. By "enhanced efficacy" salt is meant an antiperspirant salt which, when reconstituted as a 10% aqueous solution (or if already a solution, diluted with water to about 10% salt concentration in solution), produces an HPLC chromatogram wherein the Al is resolved into at least four distinct peaks (conveniently labeled peaks 2 (or 1+2), 3, 4 and 5), such as is shown in US 5,330,751, which is incorporated herein by reference, wherein at least 70%, preferably at least 80%, of the aluminum is contained in peaks 3 and 4, and wherein the ratio of the area under peak 4 to the area under peak 3 is at least 0.5, preferably at least 0.7, and more preferably at least 0.9 or higher. Most preferred are salts which exhibit an HPLC peak 4 to peak 3 area ratio of at least 0.7 when measured within two hours of preparation. Especially preferred are salts wherein at least 30%, more preferably at least 40%, of the aluminum is contained in peak 4. The aluminum present in peaks 3 and 4 should be of the Al^{c} type, not Al^{b}, when analyzed by the ferron test. Enhanced efficacy aluminum chlorohydrate is referred to as "ACH' " herein. Enhanced efficacy aluminum-zirconium chlorohydrate is referred to as "AZCH'" herein.

The pH of a solid particulate antiperspirant salt is measured, in accordance with the United States Pharmacopeia (USP), by dissolving the salt in water to form an aqueous solution of 15% concentration by weight, then measuring the pH with a properly standardized pH meter at 25 ± 2ºC.

The term "anhydrous", as used herein, means that the composition is substantially free of free water (excluding any water of hydration normally associated with the antiperspirant salt). The term "substantially free", as used herein, means that a material contains less than about 1%, preferably less than 0.1%, and more preferably 0% by weight of the identified substance. The term "zirconium hydroxychloride", as used herein, is intended to embrace zirconium compositions of the formula Zr(OH)_{4-b}Cl_{b} wherein b is about 0.8 to about 3.9, preferably about 1 to about 2, and is, thus, intended to embrace zirconium hydroxychloride and zirconyl oxychloride (sometimes written as ZrOCl₂).

The present invention is directed to a solid particulate antiperspirant salt comprising (or consisting essentially of, or consisting of) a mixture of an enhanced efficacy aluminum-zirconium chlorohydrex-amino acid and a neutralizing salt. This particulate antiperspirant salt, when measured as an aqueous solution at a concentration of 15% by weight, has a pH greater than 4.5 (e.g., up to about 5.5), preferably about 4.6 to about 5.3, more preferably about 4.8 to about 5.2. Preferably, the antiperspirant salt is substantially free of polyhydric alcohol (or diol) such as propylene glycol.

The enhanced efficacy aluminum-zirconium chlorohydrex-amino acid typically has the empirical formula AIₙZr(OH)_{[3n+4-m(n+1)]}(Cl)_{[m(n+1)]}-AA_{q} where n is 2.0 to 10.0, preferably 3.0 to 8.0; m is about 0.48 to about 1.11 (which corresponds to M:Cl ≅ 2.1-0.9), preferably about 0.56 to about 0.83 (which corresponds to M:Cl ≅ 1.8-1.2); q is about 0.8 to about 4.0, preferably about 1.0 to 2.0; and AA is an amino acid such as glycine, alanine, valine, serine, leucine, isoleucine, β-alanine, cysteine, β-amino-n-butyric acid, or γ-amino-n-butyric acid, preferably glycine. These salts also generally have some water of hydration associated with them, typically on the order of 1 to 5 moles per mole of salt (typically, about 1% to about 16%, more typically about 4% to about 13% by weight). These salts are generally referred to as aluminum-zirconium trichlorohydrex or tetrachlorohydrex when the Al:Zr ratio is between 2 and 6 and as aluminum-zirconium pentachlorohydrex or octachlorohydrex when the Al:Zr ratio is between 6 and 10. The term "aluminum-zirconium chlorohydrex" is intended to embrace all of these forms. The preferred aluminum-zirconium salt is aluminum-zirconium chlorohydrex-glycine.

The neutralizing salt typically will have a pH greater than 7 and will raise the pH of the aluminum-zirconium chlorohydrex salt with which it is mixed. Typically the neutralizing salt will be water soluble (which is preferred), although a neutralizing salt that is partially water soluble or that is water insoluble may be used in certain cases. For example, a partially soluble neutralizing salt that forms a colloidal suspension with aqueous aluminum-zirconium chlorohydrex may be used in the solution method (method 1) described *infra*, or an insoluble neutralizing salt may be used in the dry blend method (method 2) described *infra.* By "water soluble" is meant that the salt is substantially soluble in water (which is preferred) or it is soluble in an aqueous solution containing the aluminum-zirconium chlorohydrex salt.

The neutralizing salt may comprise a metal hydroxide or a salt (preferably a water soluble salt) of a strong base and an organic compound containing an acid group (e.g., a carboxylic acid group). Examples of suitable metal hydroxides and/or strong bases include sodium, potassium, calcium, magnesium, strontium, zinc and aluminum hydroxide, with sodium and potassium hydroxide being preferred. Examples of suitable organic compounds containing an acid group include weak acids such as acetic acid, ascorbic acid, benzoic acid, citric acid, gluconic acid, glycolic acid, lactic acid, etc., and the amino acids such as glycine, lysine, tyrosine, aminobutyric acid, etc. Thus, examples of suitable neutralizing salts include sodium hydroxide, sodium ascorbate, sodium benzoate, sodium citrate, sodium carbonate, sodium bicarbonate, sodium glyconate, sodium lactate, sodium glycinate, sodium lysinate, sodium tyrosinate, as well as the corresponding potassium, calcium, magnesium, strontium, zinc and aluminum salts. Sodium glycinate and sodium hydroxide are preferred.

The amino acid used to form the neutralizing salt need not be the same amino acid used in the aluminum-zirconium chlorohydrex, although it is preferred to use the same amino acid. Most preferably, the metal salt of an amino acid will be a metal glycinate. Typically the neutralizing salt will be derived from an alkali metal, such as sodium or potassium, or an alkaline earth metal, such as magnesium, calcium or strontium, although it may also be derived from other metals, such as zinc, provided that the amino acid salt formed is water soluble. Thus, a more preferred neutralizing salt will be a metal salt of an amino acid selected from the group consisting of sodium glycinate, potassium glycinate, magnesium glycinate, calcium glycinate, strontium glycinate, zinc glycinate and mixtures thereof.

The solid particulate enhanced efficacy antiperspirant salt of the present invention may be prepared in accordance with the following methods.

Method 1 (solution mixing). To an aqueous solution of an enhanced efficacy aluminum-zirconium chlorohydrex salt will be added a water soluble neutralizing salt to form a final combined solution. This final combined solution is then spray dried or vacuum dried to form a solid particulate antiperspirant salt. The amount of the water soluble neutralizing salt added prior to spray drying is that amount sufficient to provide the resulting solid particulate antiperspirant salt with a pH greater than 4.5, preferably about 4.6 to about 5.3, more preferably about 4.8 to about 5.2, when the salt is measured as an aqueous solution at a concentration of 15% by weight. Generally, the pH of the final dried salt will be identical, or nearly identical, to the pH of the final combined solution (diluted to 15% by weight solids) and no further adjustments are necessary. However, since in some cases the pH of the dried salt may be slightly different from the pH of the final combined solution prior to drying, depending upon the process conditions and materials employed, it may be useful to prepare a sample of dried salt from a test aliquot of solution taken from the main batch, measure the pH of the dried salt, then adjust the pH of the main batch solution as required.

Method 1(a). In a preferred embodiment of method 1, the starting solution is a 30%-55% aqueous solution of enhanced efficacy aluminum-zirconium chlorohydrate or chlorohydrex-amino acid (e.g., -gly). This solution can be made by mixing 30-55% enhanced efficacy aluminum chlorohydrate (ACH') with an appropriate amount (to achieve the desired Al:Zr molar ratio) of aqueous zirconium hydroxychloride (25-50%) with glycine (preferred) or without glycine. To this solution is added a neutralizing salt (e.g., sodium hydroxide or sodium glycinate or a mixture thereof), preferably as an aqueous solution of about 10% to about 90%, more preferably about 30% to about 70%, concentration by weight. The neutralizing salt is added to the antiperspirant salt solution with mechanical dispersion, such as homogenization, to assist in solubilizing and maintaining all the components in solution. Heat optionally may be applied during homogenization. After combining these components, the final combined solution is spray dried (preferred) or vacuum dried to form the dried particulate salt, which may then be pulverized or micronized to the desired particle size (e.g., <10µm).

Method 1(b). In a second embodiment of method 1, the starting solution is a 10%-20% aqueous solution of enhanced efficacy aluminum chlorohydrate (ACH'), to which is added (i) an appropriate amount (to achieve the desired Al:Zr molar ratio) of aqueous zirconium hydroxychloride (25-45%) with glycine (preferred) or without glycine and (ii) a neutralizing salt (e.g., sodium hydroxide or sodium glycinate or a mixture thereof), preferably as an aqueous solution of about 10% to about 90%, more preferably about 30% to about 70%, concentration by weight. This combined solution is concentrated in a vacuum evaporator to about 35%-55% concentration, then spray dried (preferred) or vacuum dried to form the dried particulate salt, which may then be pulverized or micronized to the desired particle size.

In method 1(b), solution (i) - the zirconium solution - and solution (ii) - the aqueous neutralizing salt solution - may be added in any order or simultaneously, or solutions (i) and (ii) may be first combined together. It is highly preferred, however, that the zirconium solution is first mixed with the aluminum chlorohydrate solution to form an aluminum-zirconium chlorohydrex solution, followed by the addition of aqueous neutralizing salt solution to raise the pH. Typically, the zirconium component will also include amino acid, such as glycine, to prevent polymerization of the zirconium species. Thus, upon addition of the zirconium (with amino acid) component to the enhanced efficacy aluminum chlorohydrate solution, an aqueous solution of the enhanced efficacy aluminum-zirconium chlorohydrex-amino acid is produced.

Method 2 (dry blending). An intimate mixture of a particulate enhanced efficacy aluminum-zirconium chlorohydrex salt and an appropriate amount of a particulate neutralizing salt is pulverized or micronized to a desired particle size (e.g., <10µm). The appropriate amount of neutralizing salt is that amount which will provide the final particulate salt blend with the desired pH within the range of the present invention.

The solid particulate antiperspirant salts of the present invention may be formulated into anhydrous topical antiperspirant compositions in any of the currently known forms, for example as a cream, a gel, a soft-solid, or a solid stick. Accordingly, a topical antiperspirant composition will include a perspiration reducing effective amount of an antiperspirant salt of the present invention suspended in a dermatologically acceptable anhydrous carrier, particularly a carrier comprising a silicone (e.g., cyclomethicone, dimethicone, etc.), typically at a concentration of about 6% to about 22% (USP) antiperspirant active by weight. Such a topical composition is particularly advantageous when it additionally includes a pH sensitive ingredient, such as a fragrance. By "pH sensitive" ingredient is meant that the ingredient, when included in an acidic composition, such as a conventional antiperspirant composition, will degrade somewhat during storage (particularly storage at 45ºC for three months) as evidenced by unacceptable odor or color of the composition, or by some other unacceptable characteristic of the composition, such as, for example, degradation in hardness or gel strength.

The anhydrous carrier may comprise any of the ingredients commonly utilized in the formulation of topical antiperspirant compositions. Advantageously, the carrier will comprise one or more volatile silicones, which evaporate quickly and provide a dry feel. The volatile silicones include the cyclic polydimethylsiloxanes, also known as cyclomethicones, which have from about three to about seven silicon atoms, and the linear polydimethylsiloxanes, also known as dimethicones, which have from about 2 to about 8 silicon atoms. The linear volatile silicones generally have viscosities of less than 5 cst, while the cyclic volatile silicones have viscosities under 10 cst. Mixtures of volatile silicones may be advantageously employed. When included in the carrier, the volatile silicones are typically present in an amount of about 10% to 90%, more typically about 20% to 70%, by weight.

The carrier may optionally include a liquid non-volatile emollient to improve emolliency and application aesthetics (e.g., reduced tackiness, slower dry-down, reduced drag and reduced whitening). The non-volatile emollient may be generally included in an amount of about 0% to about 25%, preferably about 2% to about 20%, more preferably about 5% to about 15%, by weight. Preferably the amount of non-volatile emollient will be less than about one-half the amount of volatile silicone present in the composition, and more preferably will be less than about one-third the amount of volatile silicone. Generally, the amount of non-volatile emollient should be kept to a minimum so as not to adversely affect efficacy.

When present, the non-volatile emollient will typically have a viscosity of about 5 to about 1000 cst, preferably about 10 to 500 cst. Examples of non-volatile emollients include the non-volatile silicones, typically polyalkylsiloxanes such as dimethicone (e.g. DC 200) and polyalkylarylsiloxanes such as phenyltrimethicone (e.g. DC 556), paraffinic hydrocarbons such as mineral oil and hydrogenated polyisobutene, aliphatic alcohols such as octyldodecanol, fatty alcohol esters such as C₁₂₋₁₅ alcohols benzoate and myristyl octanoate, fatty acid esters such as isopropyl palmitate, myristyl myristate and octyl isononanoate, dicarboxylic acid esters such as diisopropyl sebacate, polyethylene glycols and polypropylene glycols such as PEG-40 and PPG-20, polyethylene and/or polypropylene glycol ethers of C₄₋₂₀ alcohols such as PPG-10 butanediol, PPG-14 butyl ether, PPG-5-Buteth-7, PPG-3-Myreth-3, and Steareth-20, and polyethylene and/or polypropylene glycol esters of C₄₋₂₀ acids such as PEG-8 Distearate and PEG-10 Dioleate. Preferred emollients include the ethoxylated and propoxylated ethers and esters of C₄₋₂₀ alcohols and acids. Of course, more than one emollient may be used.

The carrier may include waxes such as fatty alcohols, for example, stearyl alcohol, cetyl alcohol, and myristyl alcohol, fatty amides, for example, Stearamide MEA and Lauramide DEA, hydrogenated castor oil (castor wax), silicone wax and polyethylene homopolymer, gelling agents such as 12-hydroxystearic acid (including esters and amides thereof) and glyceryl tribehenate, N-acyl amino acid amides such as N-lauroyl-L-glutamic acid-din-butyl amide and alkyl amides such as 2-dodecyl-N,N'-dibutylsuccinamide, thickeners such as silicone latex or silicone elastomer, suspending agents such as clays (e.g. quaternium-18 hectorite) and silicas, and fillers such as talc, polyolefins and modified corn starch.

Naturally, of course, the antiperspirant composition will also ideally include a fragrance. Because topical compositions formulated with the antiperspirant salts of the present invention are less acidic than prior art compositions, many pH sensitive fragrance components may be included that could not be used in compositions containing conventional antiperspirant salts.

The foregoing list of materials is by way of example only and is not intended to be a comprehensive list of all potential materials that may be useful in an antiperspirant composition. Obviously, the skilled worker may select materials that provide the desired application and aesthetic characteristics of the particular form of antiperspirant composition to be produced.

The present invention also embraces a method of inhibiting or reducing perspiration by topically applying an effective amount of an anhydrous antiperspirant composition as described herein to the skin of a human, preferably to the axilla, where such reduction in perspiration is desired. An effective amount is that amount which provides at least a 20% sweat reduction, preferably at least a 40% sweat reduction, when tested in accordance with a standard hot room thermal efficacy protocol, and most preferably that amount which reduces perspiration to a degree that is noticeable by the user. Typically, the amount of antiperspirant composition applied will range from about 0.1 gram to about 1.0 gram per axilla depending on the formulation or such amount as will deliver about 0.01 to about 0.25 gram of antiperspirant active per axilla.

The present invention may be further illustrated by the following examples in which the parts and percentages are by weight, unless otherwise indicated. In these examples, the abbreviation ACH' means enhanced efficacy 5/6 basic aluminum chlorohydrate with an Al:Cl ratio of about 1.95 and having an HPLC peak 4 to peak 3 area ratio of at least 0.7 with at least 80% of the aluminum contained in peaks 3 and 4. The ACH' is made by diluting ACH with water to form a solution of about 10% concentration, heating the dilute ACH solution at about 85°C for about 16 hours, then rapidly concentrating the ACH' by vacuum evaporation (for example, using a falling film evaporator) to a concentration of about 40% USP active and cooling to room temperature. The ACH' should preferably be used shortly after preparation in order to insure that it has the desired high peak 4 to peak 3 ratio.

### Example 1

(Method 1(a)) To 1760g freshly prepared ACH' (41% USP) is added 440g zirconium hydroxychloride/glycine (ZHC/gly) solution (17.5% Zr; Zr:Cl ≅ 0.7; Zr:Gly ≅ 1.0) and 400g water while mixing at 8000 rpm (IKA T25 Basic disperser) to form an aqueous enhanced efficacy aluminum-zirconium pentachlorohydrex-gly solution. After mixing this solution for ten minutes, 169g sodium glycinate solution (50%) is slowly added (over about 30 sec.) to form a final combined solution while maintaining the agitation at 8000 rpm. The agitation speed is increased to 20,000 rpm and maintained for ten minutes, then the final combined solution is spray dried (Niro Bowen spray drier; inlet/outlet temp. = 175º/105ºC; feed rate 170 ml/min) to produce a solid particulate antiperspirant salt. This salt is further pulverized (Hosokawa 100 AFG/50) to an average particle size of 5µm. The solid particulate aluminum-zirconium pentachlorohydrex-gly antiperspirant salt (Al:Zr = 9.78:1; M:Cl = 1.67:1), when dissolved in water at 15% by weight, has a pH of 4.86.

### Examples 2 to 6

The procedure of Example 1 is repeated, but the amounts of ACH', ZHC/gly, and sodium glycinate are altered to provide the following solid particulate antiperspirant salts (M:Cl ratio is adjusted as desired by addition of HCl as necessary and/or by using a ZHC with a higher or lower Zr:Cl ratio as necessary and/or by adjusting with AlCl₃ or with 2/3, 3/4 or 5/6 basic aluminum chlorohydrate):

| | |
|---|---|
| Ex. 2: | aluminum-zirconium tetrachlorohydrex-gly (Al:Zr = 3.6:1; M:Cl = 1.39:1; pH = 4.6) |
| Ex. 3: | aluminum-zirconium trichlorohydrex-gly (Al:Zr = 3.6:1; M:Cl = 1.51:1; pH = 4.7) |
| Ex. 4: | aluminum-zirconium octachlorohydrex-gly (Al:Zr = 9.6:1; M:Cl = 1.26:1; pH = 4.9) |
| Ex. 5: | aluminum-zirconium tetrachlorohydrex-gly (Al:Zr = 3.6:1; M:Cl = 1.41:1; pH = 5.1) |
| Ex. 6: | aluminum-zirconium pentachlorohydrex-gly (Al:Zr = 9.17:1; M:Cl = 1.65:1; pH = 5.0) |

The enhanced efficacy antiperspirant salts of Examples 1 to 6 look whiter and smell cleaner (i.e., no off-odor) than conventional enhanced efficacy antiperspirant salts without any significant difference in antiperspirant efficacy. Topical compositions containing these salts have no odor to interfere with added fragrances and provide greater fragrance stability.

### Example 7

The procedure of Example 1 is repeated, but with 1749g ACH', 451g ZHC/gly, 400g water and 56.5g sodium hydroxide (in place of sodium glycinate) to produce a solid particulate pentachlorohydrex-gly (Al:Zr = 9.34:1; M:Cl = 1.67:1) that, when dissolved in water at 15% by weight, has a pH of 4.61.

### Examples 8 to 17

The procedure of Example 1 is repeated, but the aqueous sodium glycinate is replaced by an appropriate amount of the following neutralizing salt solutions: potassium glycinate, magnesium glycinate, calcium glycinate, strontium glycinate, zinc glycinate, sodium alanate, sodium valinate, sodium serinate, sodium leucinate and sodium carbonate.

### Example 18

(Method 1(b)) To 1749g freshly prepared ACH' (10% USP) is added 113g zirconium hydroxychloride/glycine (ZHC/gly) solution (17.5% Zr; Zr:Cl ≅ 0.7; Zr:Gly ≅ 1.0) while mixing with a conventional laboratory agitator. To this solution is added 51.4g sodium glycinate solution (50%) with continuous mixing. This solution is evaporated to about 45% concentration, then spray dried and pulverized to produce a solid particulate pentachlorohydrex-gly (Al:Zr = 9.6:1; M:Cl = 1.67:1) that, when dissolved in water at 15% by weight, has a pH of 5.3.

### Example 19

(Method 2) A mixture of 8165g of particulate enhanced efficacy aluminum-zirconium pentachlorohydrex-gly and 907g of particulate calcium carbonate is pulverized to a particle size of 5.5 µm. This antiperspirant salt, when dissolved in water at 15% by weight, has a pH of 4.86.

### Examples 20 to 21

Two solid stick antiperspirant compositions are prepared having the following formulation: :

| Ingredient | Weight Percent |
|---|---|
| Cyclomethicone (DC 245) | 41.8 |
| AZCH'-gly | 23.0* |
| Stearyl alcohol | 17.8 |
| PPG-14 butyl ether | 11.0 |
| Hydrogenated castor wax | 2.8 |
| Myristyl myristate | 1.9 |
| Silica (R972 & A300) | 0.9 |
| Fragrance | 0.8 |

| | |
|---|---|
| * Salt of Ex. 6 (pH 5.0) at approximately 18% USP active | |

Each composition includes a fragrance - namely, pettigrain or ionone alpha - generally considered unstable under the acidic conditions present in a conventional antiperspirant solid stick. Each composition has acceptable odor and color - i.e., the fragrance is stable - when stored for three months at 45ºC. This suggests that the palette of fragrances available for inclusion in topical compositions of the present invention is substantially greater than for conventional antiperspirant compositions.

## Claims

1. A solid particulate antiperspirant salt comprising a mixture of an enhanced efficacy aluminum-zirconium chlorohydrex-amino acid and a neutralizing salt, wherein the antiperspirant salt, when measured as an aqueous solution at a concentration of 15% by weight, has a pH greater than 4.5, and wherein the antiperspirant salt is free of polyhydric alcohol.

2. The antiperspirant salt of claim 1 wherein the enhanced efficacy aluminum- zirconium chlorohydrex-amino acid comprises aluminum-zirconium chlorohydrex- glycine.

3. The antiperspirant salt of claims 1 or 2 wherein the neutralizing salt comprises a metal hydroxide, a salt of a strong base and an organic compound containing an acid group, or a mixture thereof.

4. The antiperspirant salt of claims 1 or 2 wherein the neutralizing salt comprises an alkali or alkaline earth metal hydroxide.

5. The antiperspirant salt of claims 1 or 2 wherein the neutralizing salt comprises an alkali or alkaline earth metal salt of an amino acid.

6. The antiperspirant salt of claim 2 wherein the neutralizing salt comprises an alkali or alkaline earth metal salt of glycine.

7. The antiperspirant salt of claim 2 wherein the neutralizing salt is selected from the group consisting of sodium glycinate, potassium glycinate, magnesium glycinate, calcium glycinate, strontium glycinate, zinc glycinate and mixtures thereof.

8. The antiperspirant salt of claims 1 or 2 wherein the neutralizing salt is selected from the group consisting of sodium hydroxide, sodium ascorbate, sodium benzoate, sodium citrate, sodium carbonate, sodium bicarbonate, sodium glyconate, sodium lactate, sodium glycinate, sodium lysinate, sodium tyrosinate, the corresponding potassium, calcium, magnesium, strontium and zinc salts thereof, and mixtures of two or more of these.

9. The antiperspirant salt of any of claims 1 to 8 wherein the antiperspirant salt has a pH of 4.6 to 5.3, and preferably 4.8 to 5.2.

10. The antiperspirant salt of claim 1 consisting of a spray-dried mixture of an enhanced efficacy aluminum-zirconium chlorohydrex -glycine and a water soluble neutralizing salt, wherein the antiperspirant salt, when measured as an aqueous solution at a concentration of 15% (USP) by weight, has a pH of 4.6 to 5.3.

11. The antiperspirant salt of claim 10 wherein the neutralizing salt is selected from the group consisting of sodium hydroxide, sodium ascorbate, sodium benzoate, sodium citrate, sodium carbonate, sodium bicarbonate, sodium glyconate, sodium lactate, sodium glycinate, sodium lysinate, sodium tyrosinate, the corresponding potassium, calcium, magnesium, strontium and zinc salts thereof, and mixtures of two or more of these.

12. A topical antiperspirant composition comprising a perspiration reducing effective amount of an antiperspirant salt according to any of claims 1 to 11 suspended in an anhydrous carrier.

13. The antiperspirant composition of claim 12 in the form of a cream, gel, soft-solid, or solid stick.

14. The antiperspirant composition of claims 12 or 13 additionally comprising a pH sensitive ingredient.

15. The antiperspirant composition of claim 14 wherein the pH sensitive ingredient is a fragrance.

16. A method of preparing a solid particulate antiperspirant salt according to claim 1, which method comprises providing an aqueous solution of an enhanced efficacy aluminum-zirconium chlorohydrate, adding to this solution a water soluble neutralizing salt to form a final combined solution, then spray drying the final combined solution to form a solid particulate antiperspirant salt, wherein the amount of the water soluble neutralizing salt added prior to spray drying is sufficient to provide the resulting solid particulate antiperspirant salt with a pH greater than 4.5, when the salt is measured as an aqueous solution at a concentration of 15% (USP) by weight.

17. The method of claim 16 wherein the enhanced efficacy aluminum-zirconium chlorohydrate is formed by adding zirconium hydroxychloride to an aqueous solution of an enhanced efficacy aluminum chlorohydrate in an amount to provide an Al:Zr mole ratio of 2:1 to 10:1.

18. The method of claim 17 wherein the zirconium hydroxychloride is added in the form of an aqueous zirconium hydroxychloride solution and wherein the neutralizing salt is added as an aqueous neutralizing salt solution.

19. The method of claims 17 or 18 wherein the neutralizing salt is selected from the group consisting of sodium hydroxide, sodium ascorbate, sodium benzoate, sodium citrate, sodium carbonate, sodium bicarbonate, sodium glyconate, sodium lactate, sodium glycinate, sodium lysinate, sodium tyrosinate, the corresponding potassium, calcium, magnesium, strontium and zinc salts thereof, and mixtures of two or more of these.

20. The method of any of claims 17 to 19 wherein the amount of the water soluble neutralizing salt added prior to spray drying is sufficient to provide the resulting solid particulate antiperspirant salt with a pH of 4.6 to 5.3, and preferably 4.8 to 5.2.

21. The method of any of claims 17 to 20 wherein the zirconium hydroxychloride solution is added to the aluminum chlorohydrate solution prior to the addition of the neutralizing salt solution.

22. The method of any of claims 17 to 21 wherein the zirconium hydroxychloride solution includes glycine.

23. A method of preparing a solid particulate antiperspirant salt according to claim 1, which method comprises mixing a particulate enhanced efficacy aluminum-zirconium chlorohydrex and an appropriate amount of a particulate neutralizing salt to form a mixture, then pulverizing or micronizing the mixture to form a particulate salt with a desired particle size, wherein the appropriate amount of neutralizing salt is that amount which will provide the particulate salt with a pH greater than 4.5, when the salt is measured as an aqueous solution at a concentration of 15% (USP) by weight.

24. The method of claim 23 wherein the neutralizing salt is selected from the group consisting of sodium hydroxide, sodium ascorbate, sodium benzoate, sodium citrate, sodium carbonate, sodium bicarbonate, sodium glyconate, sodium lactate, sodium glycinate, sodium lysinate, sodium tyrosinate, the corresponding potassium, calcium, magnesium, strontium and zinc salts thereof, and mixtures of two or more of these.

## Patentansprüche

1. Festes teilchenförmiges schweißhemmendes Salz, umfassend eine Mischung einer wirkverstärkten Aluminiumzirconiumchlorhydrexaminosäure und eines neutralisierenden Salzes, wobei das schweißhemmende Salz, gemessen als wässrige Lösung bei einer Konzentration von 15 Gew.-%, einen pH von mehr als 4,5 aufweist und wobei das schweißhemmende Salz frei von Polyol ist.

2. Schweißhemmendes Salz nach Anspruch 1, wobei die wirkverstärkte Aluminiumzirconiumchlorhydrexaminosäure Aluminiumzirconiumchlorhydrexglycin umfasst.

3. Schweißhemmendes Salz nach Anspruch 1 oder 2, wobei das neutralisierende Salz ein Metallhydroxid, ein Salz einer starken Base und eine organische Verbindung mit einer Säuregruppe oder eine Mischung davon ist.

4. Schweißhemmendes Salz nach Anspruch 1 oder 2, wobei das neutralisierende Salz ein Alkali- oder Erdalkalimetallhydroxid umfasst.

5. Schweißhemmendes Salz nach Anspruch 1 oder 2, wobei das neutralisierende Salz ein Alkali- oder Erdalkalimetallsalz einer Aminosäure umfasst.

6. Schweißhemmendes Salz nach Anspruch 2, wobei das neutralisierende Salz ein Alkali- oder Erdalkalimetallsalz von Glycin umfasst.

7. Schweißhemmendes Salz nach Anspruch 2, wobei das neutralisierende Salz ausgewählt ist aus der Gruppe bestehend aus Natriumglycinat, Kaliumglycinat, Magnesiumglycinat, Calciumglycinat, Strontiumglycinat, Zinkglycinat und Mischungen davon.

8. Schweißhemmendes Salz nach Anspruch 1 oder 2, wobei das neutralisierende Salz ausgewählt ist aus der Gruppe bestehend aus Natriumhydroxid, Natriumascorbat, Natriumbenzoat, Natriumcitrat, Natriumcarbonat, Natriumhydrogencarbonat, Natriumglyconat, Natriumlactat, Natriumglycinat, Natriumlysinat, Natriumtyrosinat, den entsprechenden Kalium-, Calcium-, Magnesium-, Strontium- und Zinksalzen davon und Mischungen von zwei oder mehr von diesen.

9. Schweißhemmendes Salz nach einem der Ansprüche 1 bis 8, wobei das schweißhemmende Salz einen pH-Wert von 4,6 bis 5,3 und vorzugsweise 4,8 bis 5,2 aufweist.

10. Schweißhemmendes Salz nach Anspruch 1, bestehend aus einer sprühgetrockneten Mischung eines wirkverstärkten Aluminiumzirconiumchlorhydrexglycins und eines wasserlöslichen neutralisierenden Salzes, wobei das schweißhemmende Salz, gemessen als wässrige Lösung in einer Konzentration von 15 Gew.-% (USP), einen pH-Wert von 4,6 bis 5,3 aufweist.

11. Schweißhemmendes Salz nach Anspruch 10, wobei das neutralisierende Salz ausgewählt ist aus der Gruppe bestehend aus Natriumhydroxid, Natriumascorbat, Natriumbenzoat, Natriumcitrat, Natriumcarbonat, Natriumhydrogencarbonat, Natriumglyconat, Natriumlactat, Natriumglycinat, Natriumlysinat, Natriumtyrosinat, den entsprechenden Kalium-, Calcium-, Magnesium-, Strontium und Zinksalzen davon und Mischungen von zwei oder mehr von diesen.

12. Topische schweißhemmende Zusammensetzung, umfassend eine zum Reduzieren der Schweißbildung wirksame Menge eines schweißhemmenden Salzes nach einem der Ansprüche 1 bis 11, das in einem wasserfreien Träger suspendiert ist.

13. Schweißhemmende Zusammensetzung nach Anspruch 12 in der Form einer Creme, eines Gels, eines weichen Feststoffs oder eines festen Stifts.

14. Schweißhemmende Zusammensetzung nach Anspruch 12 oder 13, die zusätzlich einen pH-empfindlichen Inhaltsstoff umfasst.

15. Schweißhemmende Zusammensetzung nach Anspruch 14, wobei der pHempfindliche Inhaltsstoff ein Duftstoff ist.

16. Verfahren zum Herstellen eines festen teilchenförmigen schweißhemmenden Salzes nach Anspruch 1, wobei das Verfahren das Bereitstellen einer wässrigen Lösung eines wirkverstärkten Aluminiumzirconiumchlorhydrats, das Zugeben eines wasserlöslichen neutralisierenden Salzes zu dieser Lösung, um eine letztendliche kombinierte Lösung zu bilden, und dann das Sprühtrocknen der letztendlichen kombinierten Lösung, um ein festes teilchenförmiges schweißhemmendes Salz zu bilden, umfasst, wobei die Menge des vor der Sprühtrocknung zugegebenen wasserlöslichen neutralisierenden Salzes ausreicht, um dem resultierenden festen teilchenförmigen schweißhemmenden Salz einen pH-Wert von mehr als 4,5 zu verleihen, wenn das Salz als wässrige Lösung in einer Konzentration von 15 Gew.-% (USP) gemessen wird.

17. Verfahren nach Anspruch 16, wobei das wirkverstärkte Aluminiumzirconiumchlorhydrat durch Zugabe von Zirconiumhydroxychlorid zu einer wässrigen Lösung eines wirkverstärkten Aluminiumchlorhydrats in einer Menge zum Bereitstellen eines Al:Zr-Molverhältnisses von 2:1 bis 10:1 gebildet wird.

18. Verfahren nach Anspruch 17, wobei das Zirconiumhydroxychlorid in der Form einer wässrigen Zirconiumhydroxychloridlösung zugegeben wird und wobei das neutralisierende Salz als wässrige neutralisierende Salzlösung zugegeben wird.

19. Verfahren nach Anspruch 17 oder 18, wobei das neutralisierende Salz ausgewählt wird aus der Gruppe bestehend aus Natriumhydroxid, Natriumascorbat, Natriumbenzoat, Natriumcitrat, Natriumcarbonat, Natriumhydrogencarbonat, Natriumglyconat, Natriumlactat, Natriumglycinat, Natriumlysinat, Natriumtyrosinat, den entsprechenden Kalium-, Calcium-, Magnesium-, Strontium- und Zinksalzen davon und Mischungen von zwei oder mehr von diesen.

20. Verfahren nach einem der Ansprüche 17 bis 19, wobei die Menge des vor der Sprühtrocknung zugegebenen wasserlöslichen Salzes ausreicht, um dem resultierenden festen teilchenförmigen schweißhemmenden Salz einen pH-Wert von 4,6 bis 5,3 und vorzugsweise 4,8 bis 5,2 zu verleihen.

21. Verfahren nach einem der Ansprüche 17 bis 20, wobei die Zirconiumhydroxychloridlösung vor der Zugabe der Lösung des neutralisierenden Salzes zu der Aluminiumchlorhydratlösung zugegeben wird.

22. Verfahren nach einem der Ansprüche 17 bis 21, wobei die Zirconiumhydroxychloridlösung Glycin enthält.

23. Verfahren zum Herstellen eines festen teilchenförmigen schweißhemmenden Salzes nach Anspruch 1, wobei das Verfahren das Mischen eines teilchenförmigen wirkverstärkten Aluminiumzirconiumchlorhydrex und einer geeigneten Menge eines teilchenförmigen neutralisierenden Salzes, um eine Mischung zu bilden, dann das Pulverisieren oder Mikronisieren der Mischung, um ein teilchenförmiges Salz mit einer gewünschten Teilchengröße zu bilden, umfasst, wobei die geeignete Menge von neutralisierendem Salz die Menge ist, die dem teilchenförmigen Salz einen pH-Wert von mehr als 4,5 verleiht, wenn das Salz als wässrige Lösung in einer Konzentration von 15 Gew.-% (USP) gemessen wird.

24. Verfahren nach Anspruch 23, wobei das neutralisierende Salz ausgewählt wird aus der Gruppe bestehend aus Natriumhydroxid, Natriumascorbat, Natriumbenzoat, Natriumcitrat, Natriumcarbonat, Natriumhydrogencarbonat, Natriumglyconat, Natriumlactat, Natriumglycinat, Natriumlysinat, Natriumtyrosinat, den entsprechenden Kalium-, Calcium-, Magnesium-, Strontium- und Zinksalzen davon und Mischungen von zwei oder mehr von diesen.

## Revendications

1. Sel antiperspirant particulaire solide comprenant un mélange d'un acide aluminium-zirconium chlorohydrex-aminé à efficacité améliorée et un sel de neutralisation, dans lequel le sel antiperspirant, lorsqu'on mesure en tant que solution aqueuse à une concentration de 15 % en poids, a un pH supérieur à 4,5, et où le sel antiperspirant est exempt d'alcool polyhydrique.

2. Sel antiperspirant selon la revendication 1, dans lequel l'acide aluminium-zirconium chlorohydrex-aminé à efficacité améliorée comprend de l'aluminium-zirconium chlorohydrex-glycine.

3. Sel antiperspirant selon les revendications 1 ou 2, dans lequel le sel de neutralisation comprend un hydroxyde métallique, un sel d'une base forte et un composé organique contenant un groupe acide, ou un mélange de ceux-ci.

4. Sel antiperspirant selon les revendications 1 ou 2, dans lequel le sel de neutralisation comprend un hydroxyde de métal alcalin ou alcalino-terreux.

5. Sel antiperspirant selon les revendications 1 ou 2, dans lequel le sel de neutralisation comprend un sel de métal alcalin ou alcalino-terreux d'un acide aminé.

6. Sel antiperspirant selon la revendication 2, dans lequel le sel de neutralisation comprend un sel de métal alcalin ou alcalino-terreux de glycine.

7. Sel antiperspirant selon la revendication 2, dans lequel le sel de neutralisation est choisi dans le groupe constitué de glycinate de sodium, glycinate de potassium, glycinate de magnésium, glycinate de calcium, glycinate de strontium, glycinate de zinc et leurs mélanges.

8. Sel antiperspirant selon les revendications 1 ou 2, dans lequel le sel de neutralisation est choisi dans le groupe constitué d'hydroxyde de sodium, ascorbate de sodium, benzoate de sodium, citrate de sodium, carbonate de sodium, bicarbonate de sodium, glyconate de sodium, lactate de sodium, glycinate de sodium, lysinate de sodium, tyrosinate de sodium, les sels correspondants de potassium, calcium, magnésium, strontium et zinc de ceux-ci, et des mélanges de deux ou plusieurs de ceux-ci.

9. Sel antiperspirant selon l'une quelconque des revendications 1 à 8, où le sel antiperspirant a un pH de 4,6 à 5,3, et de préférence 4,8 à 5,2.

10. Sel antiperspirant selon la revendication 1, constitué d'un mélange séché par atomisation d'une aluminium-zirconium chlorohydrex-glycine à efficacité améliorée et d'un sel de neutralisation hydrosoluble, où le sel antiperspirant, lorsqu'on mesure en tant que solution aqueuse à une concentration de 15 % (USP) en poids, a un pH de 4,6 à 5,3.

11. Sel antiperspirant selon la revendication 10, dans lequel le sel de neutralisation est choisi dans le groupe constitué d'hydroxyde de sodium, ascorbate de sodium, benzoate de sodium, citrate de sodium, carbonate de sodium, bicarbonate de sodium, glyconate de sodium, lactate de sodium, glycinate de sodium, lysinate de sodium, tyrosinate de sodium, les sels correspondants de potassium, calcium, magnésium, strontium et zinc de ceux-ci, et des mélanges de deux ou plusieurs de ceux-ci.

12. Composition antiperspirante topique comprenant une quantité efficace pour réduire la perspiration d'un sel antiperspirant selon l'une quelconque des revendications 1 à 11 en suspension dans un véhicule anhydre.

13. Composition antiperspirante selon la revendication 12, sous la forme d'une crème, un gel, un solide mou, ou un bâton solide.

14. Composition antiperspirante selon les revendications 12 ou 13, comprenant en outre un ingrédient sensible au pH.

15. Composition antiperspirante selon la revendication 14, dans laquelle l'ingrédient sensible au pH est un parfum.

16. Procédé de préparation d'un sel antiperspirant particulaire solide selon la revendication 1, lequel procédé comprend la fourniture d'une solution aqueuse d'un chlorhydrate d'aluminium-zirconium à efficacité améliorée, l'ajout à cette solution d'un sel de neutralisation hydrosoluble de façon à former une solution combinée finale, puis le séchage par atomisation de la solution combinée finale de façon à former un sel antiperspirant particulaire solide, dans lequel la quantité de sel de neutralisation hydrosoluble ajoutée avant le séchage par atomisation est suffisante pour fournir au sel antiperspirant particulaire solide résultant un pH supérieur à 4,5, lorsque le sel est mesuré en tant que solution aqueuse à une concentration de 15 % (USP) en poids.

17. Procédé selon la revendication 16, dans lequel le chlorhydrate d'aluminium-zirconium à efficacité améliorée est formé en ajoutant de l'hydroxychlorure de zirconium à une solution aqueuse d'un chlorhydrate d'aluminium à efficacité améliorée en une quantité pour fournir un rapport molaire Al:Zr de 2:1 à 10:1.

18. Procédé selon la revendication 17, dans lequel l'hydroxychlorure de zirconium est ajouté sous la forme d'une solution aqueuse d'hydroxychlorure de zirconium et dans lequel le sel de neutralisation est ajouté en tant que solution aqueuse de sel de neutralisation.

19. Procédé selon les revendications 17 ou 18, dans lequel le sel de neutralisation est choisi dans le groupe constitué d'hydroxyde de sodium, ascorbate de sodium, benzoate de sodium, citrate de sodium, carbonate de sodium, bicarbonate de sodium, glyconate de sodium, lactate de sodium, glycinate de sodium, lysinate de sodium, tyrosinate de sodium, les sels correspondants de potassium, calcium, magnésium, strontium et zinc de ceux-ci, et des mélanges de deux ou plusieurs de ceux-ci.

20. Procédé selon l'une quelconque des revendications 17 à 19, dans lequel la quantité du sel de neutralisation hydrosoluble ajoutée avant le séchage par atomisation est suffisante pour fournir au sel antiperspirant particulaire solide résultant un pH de 4,6 à 5,3, et de préférence 4,8 à 5,2.

21. Procédé selon l'une quelconque des revendications 17 à 20, dans lequel la solution d'hydroxychlorure de zirconium est ajoutée à la solution de chlorhydrate d'aluminium avant l'addition de la solution de sel de neutralisation.

22. Procédé selon l'une quelconque des revendications 17 à 21, dans lequel la solution d'hydroxychlorure de zirconium inclut de la glycine.

23. Procédé de préparation d'un sel antiperspirant particulaire solide selon la revendication 1, lequel procédé comprend le mélange d'un aluminium-zirconium chlorohydrex particulaire à efficacité améliorée et d'une quantité appropriée d'un sel de neutralisation particulaire de façon à former un mélange, puis la pulvérisation ou la micronisation du mélange de façon à former un sel particulaire avec une taille des particules souhaitée, dans lequel la quantité appropriée de sel de neutralisation est cette quantité qui fournira au sel particulaire un pH supérieur à 4,5, lorsque le sel est mesuré en tant que solution aqueuse à une concentration de 15 % (USP) en poids.

24. Procédé selon la revendication 23, dans lequel le sel de neutralisation est choisi dans le groupe constitué d'hydroxyde de sodium, ascorbate de sodium, benzoate de sodium, citrate de sodium, carbonate de sodium, bicarbonate de sodium, glyconate de sodium, lactate de sodium, glycinate de sodium, lysinate de sodium, tyrosinate de sodium, les sels correspondants de potassium, calcium, magnésium, strontium et zinc de ceux-ci, et des mélanges de deux ou plusieurs de ceux-ci.
